# EUROPEAN PATENT APPLICATION

(11) **EP 4 354 117 A1**
(43) Date of publication of application: **17.04.2024**
(21) Application number: 22802868.4
(22) Date of filing: 22.04.2022
(51) Int. Cl.: G01N 21/17, G01N 21/3577, G01J 3/00

(54) **SYSTEM FOR THE CONTINUOUS DETECTION AND LOGGING OF THE LEVELS OF MICROBES OF INTEREST IN WATERS**

(30) Priority: 18.05.2021 ES 202130447
(71) Applicant: Sensactive Tecnology S.L., 18193 Granada (ES)
(72) Inventor: SANCHEZ TAVIRA, Enrique, 18193 Granada (ES); IGLESIAS BAENA, Iván, 18193 Granada (ES); PROCUPETZ SCHEIN, Gabriel Edgardo, 18193 Granada (ES)
(74) Representative: Matamoron Hernandez, José Pedro
(86) International application number: PCT/ES2022/070248
(87) International publication number: WO 2022/243581

(57) **Abstract**

A system for the continuous detection of microbes of interest in waters, comprising: - at least one biosensor tank (1) having an inlet (2) and an outlet (3) for the waters to be monitored and containing nanoparticles (14) functionalised with at least one antibody specific for the antigenic capture of a particular type of microbe present in the water, and the creation of networks that give rise to an agglutination reaction that creates turbidity in the water, captured by a spectrophotometric sensor (17) or sensor for the measurement of the turbidity in the water, and - a control unit (4) for monitoring, logging, and processing the information provided by the spectrophotometric sensor (17) or sensor for the measurement of turbidity; and for monitoring the operation of the system.

## Description

### Technical sector

The present invention refers to a biotechnological system applicable to the detection and continuous and programmed monitoring of microbiological or toxic agents in aqueous medium (wastewater or fluvial, salty or fresh, potable or clean), and generally of microorganisms of interest in order to give public health or environmental responses.

The system of the invention has been developed with the aim of responding to the non-exclusive detection of SARS-CoV-2 in wastewater, although said urgent development can be specified to other types of detection (bacteria, viruses, fungi, protozoa, including xenobiotics or toxins of interest) selecting the antibody against the specific target for each case.

### State of the art

Scientific evidence has shown that the agent causative of COVID-19 (SARS-CoV-2) is a respiratory virus whose main route of exit is fecal, producing high levels of excretion from the first phase of infection, even in patients which are healthy carriers (asymptomatic) that make the detection of the virus in wastewater an appropriate indicator for the early detection of outbreaks, foci and cases.

The current detection of levels in wastewater is proving to be a useful measurement for monitoring and predicting the epidemiological status of a specific population. The current detection as a reference system is carried out through high sensitivity and specificity molecular techniques (RT-qPCR), being a test of high cost, requiring highly qualified personnel and equipment, sampling operators (with biological risk) and delay in obtaining the results of the analysis, as well as the occupation of resources with environmental samples that may be necessary for diagnosis. In addition, the detection of SARS-CoV-2 genetic material in water has limitations related to the integrity of the genetic material itself (viral RNA), which means that it cannot be detected by molecular techniques, while the viral envelope antigens are proteins resistant to the inhospitable conditions of the environment and its detection by antibodies is plausible, exceeding the limitations mentioned without requiring molecular resources.

This large-scale, real-time monitoring can reduce the need for laborious and expensive testing, by allowing efforts to be directed to areas or zones of epidemiological interest.

Consequently, it can reduce the impact of precautionary measures on people's daily lives and speed up the process by which new outbreaks are detected and controlled, thus making it a clear early warning tool for future waves of the pandemic disease.

### Presence of SARS-CoV-2 in wastewater and its relationship with early detection of COVID-19

Our society had not faced a similar health situation since 1915, when a flu pandemic devastated our country. The health emergency caused by COVID-19 and its social, economic and health impact make it necessary to search for solutions that help control the pandemic and prepare society for possible strains, viruses or new pandemics that would have devastating consequences.

Previous experiences in the use of early warning tools in wastewater have shown their utility in public health, even when the prevalence of COVID-19 presents low levels in the population.

Microbiological surveillance in wastewater can be used as an epidemiological indicator for the detection of virus circulation in the population. This detection can be carried out with adequate regularity in order to provide the information that allows the detection of possible sources of infection by carrying out population controls at the confluence of sewage pipes.

Currently, wastewater samples are taken manually, which entails the intervention of a technician-operator and a frequency of tracking with intervals between each take. Potentially contaminated water samples in hermetic containers are transported to be analyzed in specialized laboratories, with the risks and problems in current management.

All the tracking initiatives that are being carried out for the detection of COVID-19 in wastewater share the manual collection of samples and detection by PCR.

This methodology represents a series of challenges that accompany any water sampling effort, although there are three in particular which are very relevant in the environment of the COVID-19. The first challenge is the location, the second is the preservation, and the third is the integrity and safety of the exhibit.
- Location: it is essential to collect a representative water sample to ensure that the molecular analysis is reliable. It is recommended to collect samples from a turbulent environment.
- Preservation: since the analysis is not carried out immediately, samples must be preserved at 4 °C or below. Studies have shown that COVID-19 is stable for at least fourteen days if kept at these low temperatures. When collecting samples manually, it is essential to immediately place the sample on ice or in a refrigerator after collection.
- Integrity and security of the sample: once the samples have been taken, it is important to ensure their integrity when they are transferred to the analysis laboratories in a safe environment isolated from possible contamination, both from the outside to the samples and from the samples to the outside.

Consequently, handling samples manually features a series of drawbacks:
It entails a risk for the people who handle them, not only due to the possible contact with the water but also due to the aerosols that can be released in the sewers or purification centers due to the continuous movement of the water and eddies that are formed.

Not all technicians extract the water sample in the same way. Even with common operating procedures in place, it is impossible to guarantee that all samples are captured consistently.

Not all samples are collected at the proper depth.

Not all samples are collected from a location where wastewater is well mixed, and therefore, being poorly mixed, they are not representative.

Likewise, due to the manual nature of sample collection, it is difficult for all of them to be taken simultaneously (at the same time of day) at all control points.

Continuous tracking cannot be carried out due to the fact that the samples are taken discreetly, on several days, and this implies that the tracking is not consistent and in due time when the presence of COVID-19 is discovered.

### Explanation of the invention

The continuous detection and recording system of levels of relevant microbiological agents in water, object of the invention, has the aim of an instantaneous detection and continuous monitoring of the presence of microbiological (bacteria, viruses, parasites, fungi, protozoa) or toxic (xenobiotics, contaminants) agents of interest for health or for the environment, both in drinking and non-drinking water, which allows replacing the manual sample collection system and subsequent analysis in wastewater laboratories by an intelligent, automated system, without human intervention and online, both for the sampling itself, and for the evaluation and digital recording of the results of its detection analyses, which will be displayed on an ICT platform in real time.

The invention refers to an automated in situ detection system, without the need for manual sampling, sample transport and processing, or laboratory analysis, which can be adapted to environments of fresh/salt water and drinking/waste water, and performing:
- sampling and processing of the water sample by serial filtration,
- measurement by programmable turbidimetry,
- detection with specific biosensors,
- recording and interpretation with alarm system,
- data storage (Big Data) on the IoT platform in the cloud.

The system of the invention comprises at least one biosensor tank equipped with an inlet and outlet for the water to be monitored and containing nanoparticles functionalized with at least one specific antibody for the antigenic capture of a specific type of microbiological agent of interest present in water, and the creation of networks that give rise to an agglutination reaction which creates turbidity in the suspension, proportional to the captured agent.

Each biosensor tank comprises:
- water inlet and outlet filters to retain the functionalized nanoparticles inside the biosensor tank;
- a water level sensor;
- a water temperature sensor inside the biosensor tank;
- a heater in order to maintain the water at a temperature between 18 and 25 degrees Celsius
- at least one spectrophotometric sensor or a sensor for measuring the turbidity of the water to be monitored contained in the biosensor tank. Additionally, this biosensor tank can include a water pressure indicator manometer.

The system also comprises a control unit for monitoring, recording and processing the information supplied by the spectrophotometric or turbidity measurement sensor, and controlling the operation of the system.

In this system, the water sample is continuously processed and analyzed, and is accompanied by an loT platform with the ability to communicate, adjust its parameters, and analyze the sensor output.

The advantages of this system over current manual action are:
- It suits the needs of a continuous measurement system. To this aim, it includes a solution for the collection and analysis of samples in aqueous environments with possible contamination, whose measurements have epidemiological or environmental utility.
- Compatible with the procedures defined in the early warning initiatives and projects for the presence and viral load of COVID-19 in wastewater.
- Allows a rapid deployment of new units for monitoring specific outbreaks.
- Enables the automatic collection of samples at all points at specific moments in time and simultaneously.
- Ensures the protection of operators in dangerous sampling conditions. Therefore, it avoids the costs of PPE for the operators, as well as the risk involved in the manual collection of samples and their transfer.
- It represents a lower cost compared to manual collection and subsequent analysis of samples in laboratories.
- Quickness in obtaining instantaneous results in situ, with a detection and alert system.
- Allows the collection, analysis of samples and delivery of results in real time and schedules 24 hours x 7 days a week and on demand throughout the day, increasing their frequency according to the needs of viral load supervision.
- Innovative and integrated solution in Smart City and loT environments
- Smart management of wastewater.
- Automated procedures free of human intervention based on a software platform where the user can, at any time, check the levels and receive alerts in real time.
- Immediate and integrable results in the management and monitoring platforms of COVID-19 in a Smart city environment, which is compatible with IoT (Internet of Things)
- It is important to highlight that, as of the date of this document, no automated solution exists for the collection and epidemiological or environmental analysis of water on the market.

This real-time monitoring of microbiological agents in water has the ability to:
- modify its parameters, such as sensitivity, for intelligent detection,
- store the collected data of interest in microbiological or environmental records,
- transmit the results obtained in real time to an IOT platform for further processing and alarm activation.

The functionality of the system, made up of an integrated hardware/software set, means that, from the installation of the system at specific control points within sewerage routes or wastewater confluence, the status of the infection in the specific area of population is monitored.

The system provides an estimated useful life of at least 2 years for each load of nanoparticles, or until saturation of the particles due to maximum agglutination, at which time the alarm must be confirmed by PCR techniques from the same sample contained in the capture container or in a sample of new water, in order to help decision-making.

The invention has the capacity for simple detection (for a single microorganism or microbiological agent) or multiple detection (for detection of two or more microorganisms or microbiological agents), and the latter in turn can be carried out with serial detection (multisensor, with serial biosensor deposits) or simultaneous with a specific sensor (in the same container containing different particles with different antibodies, requiring a spectrometric sensor).

All the antibodies used for the functionality of the particles are antibodies of high specificity and avidity against surface epitopes of microbiological agents of interest. The preserved region or amino end of the monoclonal antibody binds to the specific surface of the nanoparticles in a stable manner, following the supplier's coating protocols.

For the total coating of the surface of the particles, appropriate blocking substances are used for each type of particle and antibody, generally being solutions of PBS with BSA and PEG 2000, which ensure the maintenance of the coating, the stability properties of the binding to the antibody, and the stability property of the particles functionalized and blocked in the aqueous suspension, which are resistant to the pH and salinity conditions of the aqueous environment to which they are going to be exposed.

The functionality of the particles and their properties with passing quality controls in the laboratory per batch of synthesis and functionalization, following the parameters and reduced sedimentation test, thermal and chemical stability (by spectrometry measurement, Bradford reaction to detect antibody release in aggressive situations and simulated pH and temperature environment) and in vitro agglutination control when exposed to their antigen (forced agglutination). The suspension of the particles is carried out in a weight/volume ratio adjusted to sensitivity criteria and within approximate values of milligrams per milliliters.

The functional adjustment of the sensitivity of the functionalized particles is carried out in the laboratory with known concentrations of antigen and/or inactivated microorganism, and obtaining a curve by turbidimetry or spectrometry in relation to the known amount of microorganisms, in order to obtain the relationship between the agglutination signal and Captured organisms, carried out by batch of functionalized particles and obtaining a semiquantitative relationship for sensitivity adjustment and graphic recording detection limits.

The monoclonal antibody specific for surface antigens and with high avidity and functionality under a wide range of pH and temperature, is covalently linked by a carboxylic bond to synthesized diameter latex or silica particles, and said saturation coating process with amino groups makes it stable and exposes the specific region to bind to the target epitope in a stable manner and giving rise to agglutination processes of aggregated particles generating turbidity in our suspension sample passage container system.

In particular, for the detection of SARS-CoV-2 in wastewater, the system integrates functionalized latex nanoparticles (200 nm in diameter) (coated beads) with a specific antibody (spike protein monoclonal antibody) that captures microbiological agents or viral particles (whole or not) present in water, through the multiple union of the particle system, creating networks that give rise to an agglutination reaction that creates turbidity, which can be determined by turbidimetry which will be continuously recorded until an alarm limit is reached.

The hydrophobic particles of latex-polystyrene or silica carbonate are synthesized in the laboratory with high purity and high quality reagents, with repeatability in the particles obtained with a homogeneous size and structure, for functionality purposes and with a coating surface of high hydrophobicity for passive coating or carboxylic surface for amino covalent binding of the coating antibody.

The suitable particles are kept in aliquots in order to confirm forced stability over time, and must always have a life of at least 2 years in suspension or until the estimated capture or agglutination limit is reached.

Once the particles are removed from the device by limit agglutination or for a duration of 2 years, said container with captured particles and microorganisms will be taken to the laboratory for verification analysis by molecular techniques (PCR for confirmation of the captured pathogen with primers or specific probes of the microorganism) and for its processing as biological waste and proper management thereof, immediately proceeding to replace the container with a new batch of functionalized particles by specialized personnel.

Any microbiological agent present in the liquid sample (bacteria, virus, fungus or yeast, cyanobacteria, protozoa) is identified in the system through specific capture and agglutination biosensors with continuous detection of turbidity or spectrometry, with the specificity of the antibody to be used and the optimal blocking conditions and detection limits to be adjusted for each system.

### Brief description of the content of the drawings

In order to complement the description that is being made and in order to facilitate the understanding of the characteristics of the invention, a set of drawings is attached to this specification in which, for illustrative and non-limiting purposes, the following has been represented:
- Figure 1 schematically shows a basic embodiment of the continuous detection and recording system of levels of microbiological agents of interest in water, according to the invention, with a biosensor tank and the control unit.
- Figure 2 is a diagram of a variant embodiment of the invention with the biosensor tank of the previous figure integrated into a water circulation circuit.
- Figure 3 shows a variant embodiment of the system with several biosensor deposits in series.
- Figure 4 shows a variant embodiment of the system with several biosensor deposits in parallel.

### Detailed description of embodiments of the invention

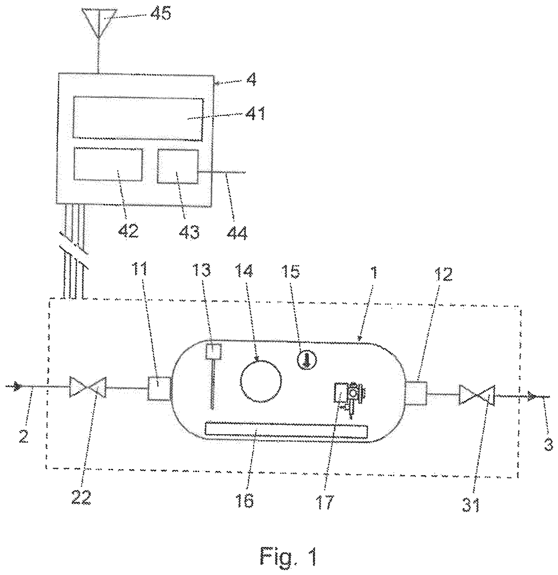

In the basic embodiment shown in Figure 1, the system comprises a biosensor tank (1), provided with an inlet (2) and an outlet (3) for the water to be monitored (which can be fresh, salt or wastewater), and of inlet (11) and outlet (12) filters for retention inside the biosensor tank of the functionalized nanoparticles (14), in the form of a stable homogeneous suspension of functionalized nanoparticles, with at least one specific antibody for the antigenic capture of a specific type of microbiological agent present in the water, and the creation of networks that give rise to an agglutination reaction that creates turbidity in the water.

The biosensor tank (1) comprises: a spectrophotometric sensor (17) or for measuring the turbidity of the water to be monitored; a water level sensor (13) to guarantee at all times that the functional nanoparticles are always immersed in water and thus avoid deterioration due to the absence of an aqueous medium; a temperature sensor (15) of the water inside the biosensor tank (1) to guarantee the correct temperature of between 18 and 25 degrees Celsius that the water must mantain for its correct reading by means of the turbidity sensor; a heater (16) for maintaining the water contained in the biosensor tank (1) at a temperature between 18 and 25 degrees Celsius.

This system comprises a control unit (4) for monitoring, recording and processing the information provided by the spectrophotometric or turbidity measurement sensor (17), and for controlling the operation of the system.

The inlet (11) and outlet (12) filters comprise a membrane disc with a hydrophobic surface and a 220nm pore size, which ensures the retention of the functionalized nanoparticles inside the container, whose size is estimated to be around 300 at 500 nm, depending on the antibody and blocking agent used.

The control unit (4) comprises: a vandal-resistant cabinet, for IP68 outdoor use, resistant to corrosive environments, water, dust, UV rays, etc., with a vandal-resistant lock; a detection unit (41) of different pathogens including COVID-19; a CPU (42) in charge of all the processing and operating sequences, as well as the loT communications with a cloud platform and the detection unit (41) of different pathogens including COVID-19; an electrical cabinet (43) for voltage input, consisting of a differential and a main switch that complies with all low voltage regulations and CE certification; an electrical supply input (44) from an electrical network, photovoltaic energy or battery, and an external antenna (45) that allows the data collected to be transmitted in real time to the loT platform by means of communication protocols such as 4G/5G/GPRS/LORA NETWORK.

This system collects and compiles the data obtained through the biosensor deposits (turbidity sensor, temperature, state of the different components that make up the device) and transmits them to the CPU unit (processing center) that allows the collection of said data. its partial processing and transmission to an IoT (Internet of Things) platform, made up of an analysis "engine" including Al in the cloud that transforms the processed data with relevant information and displays it in different formats such as tables, graphs, alarms etc.

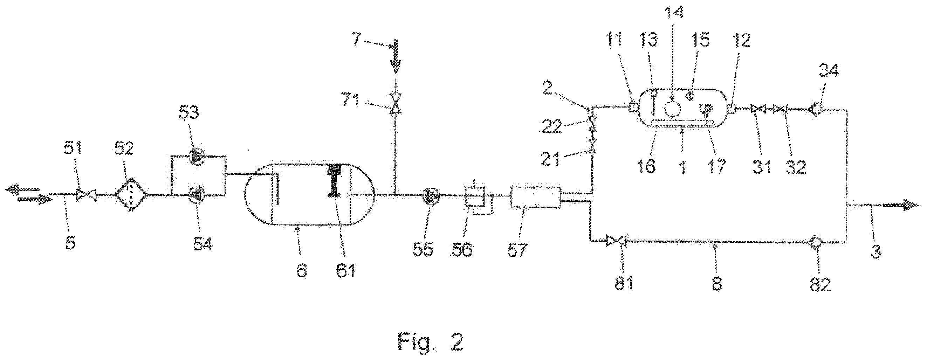

In Figure 2, the water inlet (2) to the biosensor tank (1) is connected to a wastewater inlet and outlet conduit (5) in which a valve (51) is arranged to open and close the water inlet, some filters (52) through which wastewater passes, driven by a water pump (53) to a sedimentation tank (6), which has the function of separating heavy particles from the water, such as mud, sludge, etc.

This tank (6) has a level sensor (61) that activates a water pump (55) in charge of collecting the water from the tank (6) and driving it through a pressure valve (56) and a membrane filter (57) to the inlet (2) of water to the biosensor tank (1),

Said pressure valve (56) has the function of regulating the water pressure so that it is adequate for the membrane filter (57) and the inlet filter (11) to the biosensor tank (1).

The inlet (2) and the outlet (3) of water from the biosensor tank (1) comprise two pairs of stop valves (21, 22) (31, 32) that allow the passage and shutdown of water and allow easy replacement and maintenance of the biosensor tank (1). The drainage of wastewater is carried out by circulating through the non-return valve (33) towards the water outlet (3).

The present invention works by means of a water turbidity measurement and monitoring cycle. The monitoring sequence is reprogrammable, being defined as a "repetitive measurement cycle" by definition and adjustment of the number of hours, days or months, according to the needs defined by the client and the software itself.

The monitoring sequence includes from the taking of water samples, pretreatment of the water through the filtering stages for the elimination by clarification of solid materials and contaminants in suspension, mud, algae, etc. The sequence is programmed by means of a CPU, and data transmission to the loT platform and its data processing. Each cycle is also made up of a sequence of pressure washing of the filters, tanks and pipes in order to guarantee that they do not become clogged and allow a correct measurement of turbidity.

The sequence is completed by performing a pressure washing sequence of the filters, tanks and pipes in order to ensure that they do not become clogged and to allow a correct measurement of turbidity.

This washing sequence is produced by means of a water pump (54), mounted in parallel with the water pump (53), which evacuates the water from the tank (6), and in cases where there is the possibility of connecting the device to a drinking water inlet (7) this water is made to flow through the opening of a water valve (71), a self-cleaning of the membrane filter (57) is also carried out, circulating the clean water through a pipe (8), parallel to the biosensor tank (1), provided with a water valve (81) and a non-return valve (82).

The water level sensor (13) in the biosensor tank (1) has the function of indicating that the tank is full and interrupting the operation of the water pump (55).

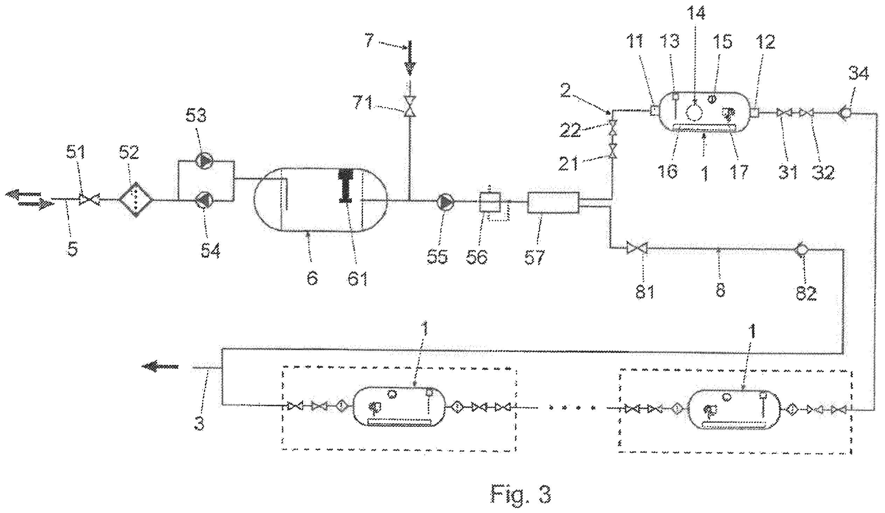

In the embodiment variant shown in Figure 3, the system comprises several biosensor tanks (1), analogous to the one described above, and arranged in series in order to allow the detection of more than one microbiological agent by arranging each sensor biosensor tank (17) of turbidity for each of the agents or microorganisms of interest to be detected.

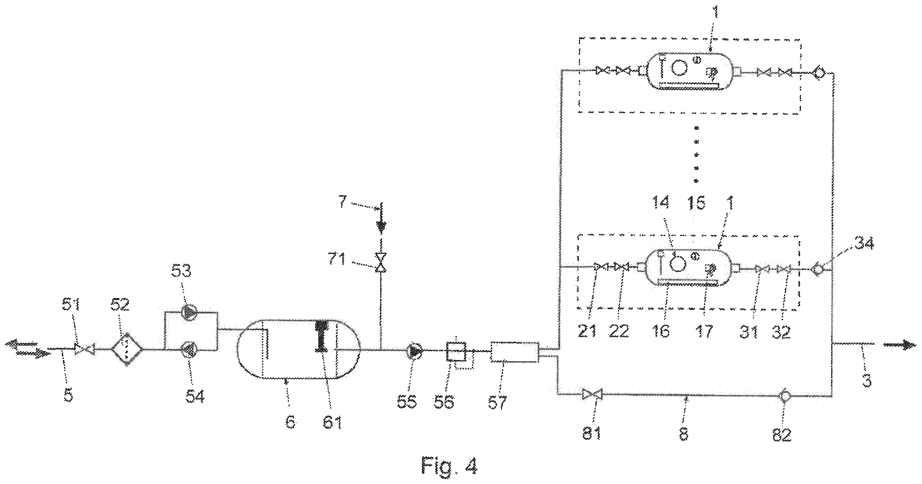

In the embodiment shown in Figure 4, the system comprises several biosensor tanks in parallel, allowing the detection of more than one microbiological or toxic agent. Each biosensor tank (1) has a turbidity sensor for each of the microorganisms or agents of interest. Each turbidity sensor records the proportional turbidity signal based on the amount of microorganism captured in each of the biosensor tanks (1).

Once the nature of the invention has been sufficiently described, as well as a preferred embodiment, it is stated for the appropriate purposes that the materials, shape, size and arrangement of the elements described may be modified, as long as this does not imply an alteration of the essential characteristics of the invention that are claimed below.

## Claims

1. System for the continuous detection of microbiological agents of interest in water; **characterized by** comprising:
- at least one biosensor tank (1) provided with an inlet (2) and an outlet (3) for the water to be monitored, and containing nanoparticles (14) functionalized with at least one specific antibody for antigen capture of a certain type microbiological agent present in the water, and the creation of networks that give rise to an agglutination reaction that creates turbidity in the water; each biosensor tank (1) comprising:
- inlet (11) and outlet (12) filters for retaining the functionalized nanoparticles (14) inside the biosensor tank (1);
- a water level sensor (13) contained in the biosensor tank (1);
- a temperature sensor (15) of the water inside the biosensor tank (1)
- a heater (16) for maintaining the water contained in the biosensor tank (1) at a temperature between 18 and 25 degrees Celsius
- at least one spectrophotometric or turbidity measurement sensor (17) of the water to be monitored contained in the biosensor tank (1) that records the proportional turbidity signal based on the amount of microorganism captured in the corresponding biosensor tank (1) and,
- a control unit (4) for monitoring, recording and processing the information provided by the spectrophotometric or turbidity measurement sensor (17), and controlling the operation of the system.

2. System, according to claim 1, **characterized in that** the inlet (11) and outlet (12) filters comprise a membrane disc with a hydrophobic surface and a pore size of around 220 nm, which ensures the retention of the functionalized nanoparticles in the interior of the container deposit, whose size is estimated to be in the order of 300 to 500 nm, depending on the antibody and blocking agent used.

3. System, according to any preceding claim; **characterized in that** the control unit (4) comprises: a detection unit (41) of different pathogens; a CPU (42) in charge of all the processing and operating sequences, and of the loT communications with a cloud platform; an electrical cabinet (43) for voltage input, a power supply input (44) and an external antenna (45) for transmitting the data collected in real time to the loT platform by means of communication protocols such as 4G/5G/GPRS/LORA NETWORK.

4. System, according to any of the preceding claims; **characterized in that** it comprises two or more biosensor tanks (1), connected in series or in parallel, between the inlet (2) and the outlet (3) of water, containing nanoparticles (14) functionalized with specific antibodies for antigen capture in each biosensor tank (1) of a certain type of microbiological agent present in the water.

5. System, according to any of the preceding claims; **characterized in that** the water inlet (2) to the biosensor tank (1) is connected to a wastewater inlet and outlet conduit (5) in which are arranged: a valve (51) for opening and closing the water inlet, filters (52) of wastewater driven by a water pump (53) to a sedimentation tank (6) equipped with a level sensor (61) that activates a water pump (55) in charge of collecting the water from the tank (6) and pushing it through a pressure valve (56) and a membrane filter (57) to the inlet (2) of water to the biosensor tank (1).

6. System, according to claim 5, **characterized in that** the inlet (2) and outlet (3) of water from the biosensor tank (1) comprise two pairs of stop valves (21, 22) (31, 32) that allow the passage and cutting wastewater.

7. System according to any of claims 5 and 6, **characterized in that** the water outlet (3) has a non-return valve (33) for passage and drainage of wastewater through said outlet (3).

8. System, according to claims 3 to 7; **characterized in that** the signals from the turbidity measurement sensors (15) provide the detection unit (41) with an identification of the agent and/or specific contaminants present in the monitored waters.

9. System, according to any of the preceding claims; **characterized in that** the functionalized nanoparticles (14) comprise functionalized latex nanoparticles (coated beads) with a monoclonal antibody that captures the microbiological agents or viral particles present in the water to be monitored, through the multiple union of the particle system, thus creating frameworks that lead to an agglutination reaction that creates turbidity in the water.
